# EUROPEAN PATENT APPLICATION

(11) **EP 1 284 287 A1**
(43) Date of publication of application: **19.02.2003**
(21) Application number: 01930008.6
(22) Date of filing: 09.05.2001
(51) Int. Cl.: C12N 15/09, C12N 7/00

(54) **METHOD OF PREPARING VIRUS VECTOR**

(30) Priority: 10.05.2000 JP 2000137302
(71) Applicant: Mitsubishi Pharma Corporation, Osaka-shi, Osaka 541-0046 (JP)
(72) Inventor: TAKASHIMA, Shigemitsu, Nat. Shikoku Cancer Center, Matsuyama-shi, Ehime 790-0007 (JP); HEIKE, Yuji, National Shikoku Cancer Center, Matsuyama-shi, Ehime 790-0007 (JP)
(74) Representative: von Kreisler, Alek, Dipl.-Chem.
(86) International application number: JP0103877
(87) International publication number: WO01085928

(57) **Abstract**

The present invention provides a means for suppressing aggregation produced after purification of viral vector. That is, the present invention provides 1) a preparation method of viral vector including at least (1) a step for purification of viral vector and (2) a step of sterilization by filtration in the presence of serum albumin of the purified viral vector obtained in the aforementioned (1); 2) a pharmaceutical composition containing viral vector and serum albumin; 3) a method for suppressing aggregation of viral vector or a method for stabilizing viral vector, which includes adding serum albumin to a viral vector-containing solution; and 4) a pharmaceutical composition containing viral vector sterilized by filtration, which is substantially free of aggregation of viral vector and/or substantially free of degradation of virus activity upon preservation in a solution state at room temperature for at least 7 days.

## Description

### Technical Field

The present invention relates to preparation of a viral vector. More particularly, this invention relates to preparation of a viral vector for a gene therapy.

### Background Art

Gene therapy aims at treatment (improvement) of the disease state of a biological organism (patient) by administering a gene as it is, or together with a suitable carrier, to the biological organism (patient) to allow expression of a protein encoded by the gene in the biological organism, and utilizing the physiological activity of the protein.

The development of a gene introduction method, which is a technique to introduce gene into cells under culture or to directly introduce gene into a biological organism, as a basic technique for a gene therapy, is indispensable. As a gene introduction method at present, a method using a virus deprived of a self-reproductive activity (viral vector), a method using a liposome, a method comprising direct injection of an expression plasmid, a method comprising introduction of gene by electric stimulation and the like have been tried. At the present moment, the viral vector method is most superior in terms of efficiency of gene introduction and efficiency of gene expression.

The viral vectors clinically applied to gene therapy so far include retroviral vector, adenoviral vector, adeno-associated viral vector, herpes viral vector, lentiviral vector and the like. Particularly, a broad range of study and development of adenoviral vector, led by US, are ongoing, since it is a useful vector.

On the other hand, preparation and purification methods of viral vector have not been established sufficiently. For example, the purification method of adenoviral vector is most commonly a method including concentration and purification by ultracentrifugation, which is followed by dialysis using a buffer and the like. To clinically apply a viral vector to a gene therapy, securing sterility for safety measures is an important factor, but dialysis is actually conducted under aseptic conditions in a clean bench and the like. However, such method is not suitable for mass preparation and is not necessarily a practical method.

### Disclosure of the Invention

To ensure sterility during preparation of a viral vector, the present inventors conceived an idea of incorporating a treatment of filter sterilization into the preparation step. However, direct sterilization by filtration of a viral vector purified by ultracentrifugation and dialysis turned out to result in a dramatically degraded virus activity. The present inventors investigated the cause and confirmed that purification causes aggregation of viral vector, and the vector cannot pass through a filter for filter sterilization.

The object of the present invention is based on such new problem and is to provide a means for suppressing aggregation of a viral vector, which is produced by the purification step.

In consideration of the above-mentioned situation, the present inventors have proceeded with the study and, as a result, found that addition of serum albumin during sterilization by filtration suppresses clogging of a filter caused by aggregation of a viral vector, thereby permitting preferable sterilization by filtration, and further found that a viral vector highly purified and superior in stability can be prepared, which resulted in the completion of the present invention.

Accordingly, the present invention relates to 1) a method for preparing a viral vector, which includes at least (1) a step of purification of a viral vector and (2) a step of sterilization of the purified viral vector, which is obtained in the aforementioned (1), by filtration in the presence of serum albumin; 2) a pharmaceutical composition comprising a viral vector and serum albumin; 3) a method of suppressing aggregation of viral vector or a method of stabilizing a viral vector, which includes adding serum albumin to a viral vector-containing solution; and 4) a pharmaceutical composition comprising a viral vector sterilized by filtration, which is substantially free of aggregation of a viral vector and/or substantially free of degradation of virus activity upon preservation in a solution state at room temperature for at least 7 days. The present invention is explained in detail in the following.

### A. Serum albumin

The origin of the serum albumin in the present invention is not particularly limited. The origin may be human or a mammal such as cow, horse, sheep, goat, rabbit, guinea pig and the like. For clinical application, one originated from human (human serum albumin, hereinafter to be referred to as HSA) is preferably used in consideration of antigenicity and the like. The serum albumin may be originated from blood or plasma and, in addition, may be prepared by a method such as cell culture, gene recombination and the like.

Examples of the serum albumin derived from blood or plasma include serum albumin itself, serum albumin-containing fraction, purified crude fraction thereof and the like. Specifically, fetal bovine serum (FBS), fraction V by Cohn's cold ethanol fractionation method, heat stable plasma protein disclosed in JP-A-50-111225 and the like, heated human plasma protein which is a commercially available preparation (plasmanate, manufactured by Bayer, Ltd.), PPF (plasma protein fraction, manufactured by Baxter Limited) and the like.

More preferable example is plasma-derived HSA. The production method thereof is known. For example, a method including removing polymer after sterilization by heating (JP-A-2-111728), a method including sterilization by heating after anion exchanger treatment or affinity chromatography treatment (JP-A-2-191226), a method including sterilization by heating after anion exchanger treatment and then cation exchanger treatment (JP-A-3-17023) and the like are mentioned. In addition, a commercially available preparation (product name: Albumin, manufactured by Welfide Corporation and the like) may be used.

As an HSA prepared by cell culture, serum albumin can be prepared using a hepatic cancer cell, such as HepG2 cell and the like.

As an HSA prepared by gene recombination, gene recombinant HSA (hereinafter to be referred to as rHSA) and the like are exemplified. While rHSA is not particularly limited as long as it is a human serum albumin produced by an HSA-producing host prepared by gene manipulation, it is preferably rHSA substantially free of contaminant components (e.g., protein etc.) derived from production host, and more preferably, rHSA harvested and purified, according to a known separation technique and purification technique, from a culture filtrate, bacterial cell or cell of an rHSA-producing host cultured by a known technique. It is also possible to use a transgenic animal or a transgenic plant (JP-A-9-509565 and JP-A-10-504289). The following methods are specifically mentioned.

The host used in the present invention for obtaining rHSA is not particularly limited as long as it is prepared by gene manipulation, and those described in known publications and those to be developed from now can be appropriately utilized. Specifically, bacteria made to be rHSA-produceable by gene manipulation (e.g., Escherichia coli, yeast, Bacillus subtilis etc.), animal cell and the like are mentioned. Particularly, yeast, preferably the genus Saccharomyces [e.g., Saccharomyces cerevisiae], or the genus Pichia [e.g., Pichia pastoris] is used as a host. In addition, auxotrophic strains and antibiotic-sensitive strains may be used. More preferably, Saccharomyces cerevisiae AH22 strain (a, his 4, leu 2, can 1) and Pichia pastoris GTS115 strain (his 4) are used.

Preparation of these rHSA-producing hosts, production method of rHSA by culturing the host, and separation and harvesting of rHSA from the culture can be done by employing a known method or a method analogous thereto. For example, the preparation method of rHSA-producing host is exemplified by a method comprising use of a conventional HSA gene (JP-A-58-56684, JP-A-58-90515, JP-A-58-150517), a method comprising use of a novel HSA gene (JP-A-62-29985, JP-A-1-98486), a method comprising use of a synthetic signal sequence (JP-A-1-240191), a method comprising use of a serum albumin signal sequence (JP-A-2-167095), a method comprising incorporation of a recombinant plasmid on a chromosome (JP-A-3-72889), a method comprising fusion of hosts to each other (JP-A-3-53877), a method comprising mutation in a methanol-containing medium, a method comprising use of a mutant AOX2 promoter (JP-A-6-90768, JP-A-4-299984), an expression of HSA by Bacillus subtilis (JP-A-62-25133), an expression of HSA by yeast (JP-A-60-41487, JP-A-63-39576, JP-A-63-74493), an expression of HSA by Pichia yeast (JP-A-2-104290) and the like.

Of these, the method comprising mutation in a methanol-containing medium comprises the following steps. That is, a plasmid having a transcriptional unit, where HSA is expressed under control of AOX1 promoter, is introduced into an AOX1 gene region of a suitable host, preferably Pichia yeast, and specifically GTS115 strain (NRRL deposit No. Y-15851) by a conventional method to give a transformant (see JP-A-2-104290). This transformant has a weak growth capability in a methanol medium. Thus, this transformant is cultured in a methanol-containing medium to induce mutation and only viable strains are recovered. The methanol concentration at this time is, for example, about 0.0001-5%. The medium may be an artificial medium or a natural medium. The culture conditions are, for example, about 15-40°C for 1-1000 hr.

The culture method of the rHSA-producing host is exemplified by, besides the method described in the above-mentioned publication, a method for obtaining high concentrations of a bacterial cell and a product, while avoiding high concentration substrate inhibition of the producing bacterial cell by supplying high concentration glucose or methanol and the like by a suitable small amount in a fed-batch culture (semi-batch culture) (JP-A-3-83595), a method for potentiating rHSA production by adding fatty acid to a medium (JP-A-4-293495) and the like.

As a method for isolating and purifying rHSA produced by culture treatment from a component derived from a host cell, culture component and the like with sufficient precision, various methods have been proposed. As a conventional method, for example, a method comprising subjecting a yeast culture medium containing rHSA to compression and ultrafiltration treatment and heat treatment and ultrafiltration treatment, and then to a step of a cation exchanger treatment, a hydrophobic chromatography treatment, an anion exchanger treatment and the like (JP-A-5-317079; Biotechnology of Blood Proteins, vol. 227, pp. 293-298, issued in 1993) and the like is exemplified. In addition, a method comprising further subjecting, after the above-mentioned conventional method, to a step of treatment using chelate resin or a treatment using boric acid or a salt thereof has been reported (JP-A-6-56883, JP-A-6-245789). Furthermore, a streamline method using an expanded bed adsorption (JP-A-8-116985) may be used after heat treatment of the yeast culture medium, and the like. The thus prepared and purified rHSA can be subjected to a treatment by a known method, such as sterilization heating, ultrafiltration treatment, addition of stabilizer, sterilization by filtration, dispensing, lyophilization and the like.

Of these, a gene recombination-derived product is more preferable, because it is free of variation in quality between lots, easy to purify, free of a risk of contamination with virus, prion and the like, and the like.

### B. Viral vector

The viral vector to be used in the present invention is not particularly limited, and known ones can be used widely. For use of the viral vector in gene therapy, for example, adenoviral vector, adeno-associated viral vector (AAV), retroviral vector, herpes viral vector, lentiviral vector and the like are mentioned. Particularly preferred in the present invention is adenoviral vector.

The gene and the gene expression system to be carried on the viral vector are not particularly limited, and known ones, namely, those using a gene encoding various proteins having a physiological activity can be widely used. LacZ (β-galactosidase), IL2 and the like are exemplified.

The purification method of a viral vector is not particularly limited, and may be known. Specific examples include ultracentrifugation, dialysis, ion exchanger treatment (EP-A-751988, EP-A-964923) and the like.

First, a suitable host·cell is infected with a viral vector (gene introduction), cultured in a medium, and the viral vector amplified and prepared in large amounts is recovered from a cell or culture medium, which is then subjected to a purification means.

An ultracentrifugation method is a step for concentration and purification of a viral vector. When an ultracentrifugation method is used, a density gradient method (multi-layer method) of cesium chloride, sucrose and the like can be utilized. The concentration condition can be determined appropriately depending on the kind of the viral vector to be separated. Specifically, when cesium chloride is used, it can be set in the range of about 1-2 g/ml or about 1-5 M. The pH condition is, for example, about 6-9. To meet such pH conditions, buffers such as HEPES buffer, Tris-hydrochloride buffer and the like can be used. As the treatment conditions of ultracentrifugation, for example, the number of rotation is about 10,000 - 50,000 rpm, temperature is about 0-10°C and the time is about 1-15 hours. Ultracentrifugation can be applied repeatedly. The recovered fraction can be determined appropriately according to the kind of the viral vector.

The dialysis method is a step for removing cesium chloride and the like from a viral vector solution for purification. When a dialysis method is used, for example, pH is about 6-9 and the salt concentration is about 0.01-1 M. To meet such pH condition, buffers such as isotonic phosphate buffer (PBS), Tris-hydrochloride buffer and the like can be used. Moreover, glycerol can be added. The concentration of glycerol is about 1-20 v/v%, preferably about 5-15 v/v%. As the dialysis treatment condition, for example, the temperature is about 0-10°C, the time is about 1-10 hours. Dialysis can be applied repeatedly.

A purified viral vector can be obtained by the above-mentioned purification step.

### C. Sterilization by filtration

The preparation method of the viral vector of the present invention is characterized in that the purified viral vector obtained in the above-mentioned B is sterilized by filtration in the presence of serum albumin. Specifically, serum albumin is added to a solution of a purified viral vector obtained by the aforementioned method, which is followed by sterilization by filtration.

The solution of the purified viral vector used is prepared to about 10¹⁰-10¹² pfu/ml. The concentration of the serum albumin to be added is, for example, about 0.5-10 w/v%, preferably about 1-5 w/v%. This solution has, for example, a pH of about 6-9 and a salt concentration of about 0.01-1 M. To meet such pH condition, buffers such as isotonic phosphate buffer (PBS), Tris-hydrochloride buffer and the like can be used. Moreover, glycerol can be added. The concentration of glycerol is about 1-20 v/v%, preferably about 5-15 v/v%.

Sterilization by filtration is generally conducted after a predetermined amount of serum albumin is added to a solution of purified viral vector, and incubated at 0-10°C for 5-60 min, preferably 15-30 min. These treatment conditions are appropriately set depending on the degree of purification, concentration of the viral vector to be used, concentration of serum albumin, and the like. In the event aggregation of the viral vector is not confirmed, it is also possible to apply filter sterilization treatment immediately after addition of serum albumin.

The filter to be used for sterilization by filtration may be one having a pore size of about 0.01-1 µm, preferably about 0.1-0.5 µm. It is also possible to use a commercially available filter. Specific examples thereof include a filter for sterilization by filtration having a pore size of 0.22 µm (product name: Millex-GS, manufactured by Millipore Corporation) and the like.

### D. Properties of the obtained highly purified viral vector

A viral vector-containing solution prepared by the aforementioned method (hereinafter to be simply referred to as a viral vector solution) does not substantially cause aggregation, maintains high titer (also referred to as "highly purified"), and is sterilized by filtration. It is also superior in preservation stability.

The viral vector solution has a highly purified virus activity of about 10¹⁰-10¹² pfu/ml. The activity can be determined by a biological measurement method using a producer cell (e.g., 293 cells and the like), absorbance method and the like (see Jpn. J. Med. Sci. Biol., vol. 47, pp. 157-166 (issued in 1994) and the like).

In the present specification, by "does not cause aggregation substantially" is meant absence of aggregation having a size of not less than the pore size (about 0.01-1 µm, preferably 0.1-0.5 µm) of the filter to be used for sterilization by filtration, or if present, it is present only in a small amount. When such aggregation is produced, it does not pass through a filter but is trapped. The presence or otherwise of aggregation can be directly determined or confirmed using an electron microscope, or indirectly confirmed by examining whether or not the viral vector passes through the filter for sterilization by filtration.

The viral vector solution becomes preservable in a solution state by adding serum albumin. In other words, the virus activity can be maintained fine. The concentration of serum albumin to be added is, for example, about 0.5-10 w/v%, preferably 1-5 w/v%. This solution has, for example, a pH of about 6-9 and a salt concentration of about 0.01-1 M. To meet such pH condition, a buffer such as isotonic phosphate buffer (PBS), Tris-hydrochloride buffer and the like can be used. In addition, glycerol can be added. The concentration of glycerol is about 1-20 v/v%, preferably about 5-15 v/v%. It is also possible to use a known stabilizer as necessary. For example, sugar, amino acid, organic acid and the like are mentioned (WO 99/61063). The viral vector solution can be preserved in a solution state as mentioned above, but can be also preserved according to a conventionally known method such as freezing, lyophilization and the like.

The present invention provides a pharmaceutical composition comprising viral vector and serum albumin, particularly a pharmaceutical composition substantially free of aggregation of viral vector, which is sterilized by filtration. As used herein, the "substantially free of aggregation of viral vector" means the same as the above-mentioned "does not cause aggregation substantially". This pharmaceutical composition is stable during preservation and, specifically, substantially free of degradation of the activity upon preservation at room temperature for at least 7 days. By the "room temperature" is meant the temperature range generally employed in this field, which is specifically about 10-30°C. By the "substantially free of degradation of virus activity" is meant the retention of vector function at a level sufficient for the object of use, such as gene introduction for gene therapy and gene expression. More specifically, for example, it means the state where virus activity (pfu/ml) remains at a level of at least 85% (100% before preservation) when preserved at room temperature for at least 7 days.

The pharmaceutical composition of the present invention is provided in an orally or parenterally suitable form as a pharmaceutical composition obtained by mixing a viral vector and serum albumin with a pharmaceutically acceptable carrier (excipient, binder, disintegrant, flavoring agent, odoring agent, emulsifying agent, diluent, dissolution aids and the like), or as a preparation such as tablet, pill, powder, granule, capsule, troche, syrup, liquid, emulsion, suspension, injection (liquid, suspension and the like), suppository, inhalant, transdermally absorbing agent, eye drop, eye ointment and the like. As the pharmaceutically acceptable carrier and the method for preparation into each dosage form, those generally used or practiced in this field can be utilized.

The amount of the viral vector to be contained in these pharmaceutical compositions may vary and is determined depending on the object thereof, various factors of vector such as the kind of vector, the kind of the gene to be introduced and the like, as well as condition, body weight, age and the like of the recipient patient. The amount of the serum albumin to be contained in the pharmaceutical composition is not particularly limited as long as it can achieve the object of preventing aggregation of the coexistent viral vector, and it does not adversely affect the biological organism. When the pharmaceutical composition is specifically a liquid preparation, for example, serum albumin is contained at a concentration of about 0.5-10 w/v%, preferably about 1-5 w/v%, when the concentration of viral vector is 10¹⁰-10¹² pfu/ml.

### Examples

The present invention is explained in detail by referring to Examples and Experimental Examples, which are not to be construed as limitative.

### Example 1

Based on the aforementioned Jpn. J. Med. Sci. Biol., vol. 47, pp. 157-166 (issued in 1994), adenoviral vector (AxCA-LacZ) that expresses LacZ was prepared. This viral vector has a CAG promoter. 293 cells were infected with the prepared viral vector and the viral vector produced intracellularly and in the culture medium was released by repeated freeze-thawing and recovered. This was subjected to a purification treatment.

First, the viral vector was separated and purified by an ultracentrifugation treatment. 4 M Cesium chloride/10 mM HEPES buffer (pH 7.4) and 2.2 M cesium chloride/10 mM HEPES buffer (pH 7.4) were layered in a centrifugal tube (Ultra-Clear TM tubes, manufactured by Beckman), and a viral vector solution was layered thereon, which was subjected to ultracentrifugation at 4°C, 25000 rpm for 3-4 hr. The ultracentrifugation apparatus used was Optima L-70K (manufactured by Beckman). A fraction of viral vector was recovered from the intermediate layer thereof. Again, 4M cesium chloride/10 mM HEPES buffer (pH 7.4) and 2.2 M cesium chloride/10 mM HEPES buffer (pH 7.4) were layered in a centrifugal tube, and a solution of viral vector was layered thereon, which was subjected to ultracentrifugation at 4°C, 35000 rpm overnight. A fraction of viral vector was recovered from the intermediate layer thereof.

Then, cesium chloride was removed from the viral vector solution by a dialysis treatment. That is, the recovered viral vector solution was dialyzed at 4°C for 4 hr using regenerated cellulose (molecular weight cut-off of 3500, product name: Bio-tech dialysis cup, manufactured by Bio-tech International Inc.) as a dialysis membrane and 10 v/v% glycerol-containing isotonic phosphate buffer (PBS, pH 7) as an outer chamber solution. The outer chamber solution was exchanged to a fresh solution and the dialysis treatment was repeated.

rHSA prepared in Reference Example (mentioned below) was added to the obtained viral vector solution (concentration of 10¹² pfu/ml) to a final concentration of 1 w/v%, left standing at 4°C for 30 min and sterilized by filtration. Using a filter (pore size 0.22 µm) for sterilization by filtration (product name: Millex-GS, manufactured by Millipore Corporation), the mixture was sterilized by filtration to give a viral vector highly purified and sterilized by filtration.

### Example 2

Ultracentrifugation was performed as in the following. That is, 1.4 g/ml cesium chloride/50 mM Tris-hydrochloride buffer (pH 8.1) and 1.2 g/ml cesium chloride/50 mM Tris-hydrochloride buffer (pH 8.1) were layered on a centrifugal tube and a viral vector solution was layered thereon, which was subjected to ultracentrifugation at 4°C, 18000 rpm for 5 hr. A fraction of viral vector was recovered from the intermediate layer thereof. Again, 1.4 g/ml cesium chloride/50 mM Tris-hydrochloride buffer (pH 8.1) was layered in a centrifugal tube, and a solution of the viral vector was layered thereon, which was subjected to ultracentrifugation at 4°C, 25000 rpm for not less than 12 hr. A fraction of viral vector was recovered from the intermediate layer thereof.

Except this operation, all other steps were done in the same manner as in Example 1 to give a viral vector highly purified and sterilized by filtration in the same manner.

### Example 3

Completely in the same manner as in Example 1 except that an adenoviral vector (AxCA-hIL2; the same vector as in Example 1 except that LacZ gene was substituted by hIL2 gene) that expresses human IL2 was used, a viral vector highly purified and sterilized by filtration was prepared in the same manner.

### Experimental Example 1

The aggregation suppressing effect on viral vector by rHSA was confirmed. The level of aggregation produced when rHSA was added to a viral vector solution obtained by ultracentrifugation and dialysis according to Example 1 to a final concentration of 1 w/v% and that when the solution was not added were compared. The level of aggregation was evaluated by observation using a transmission electron microscope (x29000-100000 magnification) at 30 min after addition of rHSA. The results are shown in Table 1.

**Table 1**

| | Level of aggregation |
|---|---|
| without addition of rHSA | +++ |
| with addition of rHSA | - |

In the table, - means that aggregation was scarcely observed and +++ means that aggregation was easily observed.

### Experimental Example 2

The level of filter passage obstruction due to aggregation was compared by indirectly determining the virus activities before and after sterilization by filtration. A LacZ expression viral vector was prepared according to Example 1, and a viral vector in the viral vector solution before sterilization by filtration, that in the filtrate after sterilization by filtration and that in a filtered fraction (non-passage fraction) were infected. The amount of a protein expressed (β-galactosidase) was measured and used as an index of virus activity.

The measurement was performed according to the following method. Each sample (5 µl, viral vector solution) was added to Hela cell. After infection and culture under given conditions, the medium (solvent) was removed and washed with PBS(-). Ice-cooled 0.24 w/v% glutaraldehyde/PBS was added by 1.5 ml each and, after fixing at 4°C for 5 min, the solvent was removed. A 5-bromo-4-chloro-3-indolyl β-galactopyranoside (hereinafter to be referred to as X-gal) solution (consisting of 5 mM potassium ferrous cyanide, 5 mM potassium ferric cyanide, 2 mM magnesium chloride and 0.2 mg/ml of X-gal) was added and the mixture was reacted at 36°C for 4 hr. After staining, the stained part was visually observed and used as an index of virus activity. That is, because the virus is present and the cell infected with the virus expresses LacZ, it is stained with X-gal solution. As a control, a system without addition of rHSA was also subjected to the same experiment. The results are shown in Table 2.

**Table 2**

| | Degree of staining | | |
|---|---|---|---|
| | before sterilization by filtration | filtrate | filtered fraction |
| without addition of rHSA | +++ | - | +++ |
| with addition of rHSA | +++ | +++ | +/- |

In the table, +++ means that extremely strong staining was observed, +/- means that certain level of staining was observed and - means that staining was not observed.

When rHSA was not added, the viral vector could not pass through the filter for sterilization by filtration due to aggregation thereof, and was trapped in the filtered fraction. In contrast, when rHSA was added, the viral vector almost completely passed through the filter, and it was confirmed that sterilization by filtration was possible without causing clogging of the filter due to aggregation.

### Experimental Example 3

The suppressive effect on the aggregation of viral vector was confirmed using various serum albumins and fractions thereof. As a sample, FBS, bovine serum albumin (BSA), plasma-derived HSA or rHSA was added and the experiment was conducted according to Experimental Example 2. The degree of obstruction on passage during sterilization by filtration was evaluated from the level of staining. The results are shown in Table 3.

**Table 3**

| | Degree of staining | | |
|---|---|---|---|
| | before sterilization by filtration | filtrate | filtered fraction |
| FBS | +++ | +++ | +/- |
| BSA | +++ | +++ | +/- |
| Plasma-derived HSA | +++ | +++ | +/- |
| rHSA | +++ | +++ | +/- |

Each symbol in the Table is as defined in Table 2.

In every case, the viral vector passed through the filter and aggregation was not observed. The result of sterilization by filtration was fine.

### Experimental Example 4

The level of stabilization of a viral vector solution by serum albumin was confirmed. rHSA and glycerol/PBS were added to the purified viral vector solution prepared in Example 1 to give a viral vector solution having concentrations of viral vector 10¹² pfu/ml, rHSA 1 w/v% and glycerol 10 v/v%. The solution was preserved at room temperature (15-25°C) for 7 days, and the virus activity in the solution was measured with the lapse of time. As a control, a viral vector solution without rHSA was also subjected to the same experiment. The virus activity was determined according to Experimental Example 2. The results are shown in Table 4.

**Table 4**

| | Degree of staining | | | |
|---|---|---|---|---|
| | before preservation | day 1 of preservation | day 3 of preservation | day 7 of preservation |
| without addition of rHSA | +++ | +++ | - | - |
| with addition of rHSA | +++ | +++ | +++ | +++ |

Each symbol in the Table is as defined in Table 2.

It was found that the viral vector prepared by the method of the present invention was stable in a solution state at room temperature for at least 7 days by the addition of rHSA.

### Experimental Example 5

Using a system for directly determining the virus activity, the same experiment as Experimental Example 2 was conducted. The method for determining the virus activity followed the aforementioned Jpn. J. Med. Sci. Biol., vol. 47, pp. 157-166 (issued in 1994). The results are shown in Table 5.

**Table 5**

| | virus activity (pfu/ml) | |
|---|---|---|
| | before sterilization by filtration | filtrate |
| without addition of rHSA | 1.64x10¹¹ | 0 |
| with addition of rHSA | 1.01x10¹¹ | 8.61x10¹⁰ |

From the above results, it was found that addition of rHSA during sterilization by filtration of a viral vector enabled recovery of most of the viral vector from the filtrate. In other words, this viral vector was confirmed to be sufficiently capable of passing through a filter for sterilization by filtration.

### Experimental Example 6

The level of filter passage obstruction due to aggregation was compared by indirectly determining the virus activities before and after sterilization by filtration. A viral vector expressing hIL2 was prepared according to Example 3, cells were infected with a viral vector in the viral vector solution before sterilization by filtration or that in the filtrate after sterilization by filtration, and the amount of an expressed protein (hIL2) was measured and used as an index of virus activity.

The measurement was performed according to the following method. Each sample (5 µl, viral vector solution) was added to Hela cell. After infection and culture under given conditions, the medium (solvent) was recovered and the amount of hIL2 in the medium was determined by the ELISA method and used as an index of virus activity. That is, the fact that a cell infected with the virus expresses hIL2 was utilized. As a control, a system without addition of rHSA was also subjected to the same experiment. The results are shown in Table 6.

**Table 6**

| | hIL-2 concentration (pg/ml) | |
|---|---|---|
| | before sterilization by filtration | filtrate |
| without addition of rHSA | 3872±301 | 168±66 |
| with addition of rHSA | 3699±402 | 3409±514 |

The values in the table show average ± standard deviation.

From this result, it was found that addition of rHSA during sterilization by filtration of a viral vector enabled recovery of most of the viral vector from the filtrate. In other words, this viral vector was confirmed to be sufficiently capable of passing through a filter for sterilization by filtration.

### Reference Example

As rHSA, one prepared and purified according to the method disclosed in JP-A-8-116985 using a HSA producing Pichia pastoris yeast was used.

### Industrial Applicability

According to the present invention, the use of serum albumin can suppress aggregation of viral vector, and therefor, affords preparation of a viral vector (solution), which can maintain high titer even after sterilization by filtration. Particularly, the significance of improved safety when applying a viral vector to a gene therapy is extremely high, which is afforded by the enabled sterilization by filtration. According to the present invention, moreover, because a viral vector can be prepared and purified efficiently in a large amount, a viral vector in a sufficient amount necessary for gene therapy can be supplied to clinical situations.

This application is based on a patent application No. 137302/2000 filed in Japan, the content of which is hereby incorporated by reference.

## Claims

1. A method for preparing a viral vector, which comprises at least the steps of
(1) purifying viral vector, and
(2) sterilizing the purified viral vector obtained in the aforementioned (1) by filtration in the presence of serum albumin.

2. The method of claim 1, wherein the viral vector is purified by at least one treatment step selected from the group consisting of ultracentrifugation, dialysis and an ion exchanger treatment.

3. The method of claim 2, wherein the ultracentrifugation is conducted under the conditions of pH 6-9, 10,000 - 50,000 rpm, temperature 0-10°C and 1-15 hours.

4. The method of claim 2, wherein the dialysis is conducted under the conditions of pH 6-9, salt concentration 0.01-1 M, temperature 0-10°C and 1-10 hours.

5. The method of claim 1, wherein the sterilization by filtration is conducted under the conditions of pH 6-9, salt concentration 0.01-1 M, using a filter having a pore size of 0.01-1 µm.

6. The method of claim 1, wherein the serum albumin has a concentration of 0.5-10 w/v%.

7. The method of claim 1, wherein the viral vector is at least one selected from among adenoviral vector, adeno-associated viral vector (AAV), retroviral vector, herpes viral vector and lentiviral vector.

8. A pharmaceutical composition comprising viral vector and serum albumin.

9. The pharmaceutical composition of claim 8, wherein the viral vector is at least one selected from among adenoviral vector, adeno-associated viral vector (AAV), retroviral vector, herpes viral vector and lentiviral vector.

10. The pharmaceutical composition of claim 8, which is a liquid preparation.

11. The pharmaceutical composition of claim 10, wherein the viral vector has a concentration of 10¹⁰-10¹² pfu/ml.

12. The pharmaceutical composition of claim 10, wherein the serum albumin has a concentration of 0.5-10 w/v%.

13. The pharmaceutical composition of claim 10, which is a solution having a pH 6-9 and a salt concentration of 0.01-1 M.

14. A method for suppressing aggregation of viral vector, which comprises adding serum albumin to a solution containing the viral vector.

15. The method of claim 14, wherein the serum albumin is added at a concentration of 0.5-10 w/v%.

16. A method for stabilizing a viral vector, which comprises adding serum albumin to a solution containing the viral vector.

17. The method of claim 16, wherein the serum albumin is added at a concentration of 0.5-10 w/v%.

18. A pharmaceutical composition comprising viral vector, which has at least one of the following characteristics:
a) sterilized by filtration,
b) substantially free of aggregation of viral vector
c) substantially free of degradation of virus activity upon preservation in a solution state at room temperature for at least 7 days.

19. A pharmaceutical composition comprising viral vector, which has all the following characteristics:
a) sterilized by filtration,
b) substantially free of aggregation of viral vector
c) substantially free of degradation of virus activity upon preservation in a solution state at room temperature for at least 7 days.

20. The pharmaceutical composition of claim 18 or 19, wherein at least 85% (100% before preservation) of the virus activity remains upon preservation in a solution state at room temperature for at least 7 days.
